# EUROPEAN PATENT APPLICATION

(11) **EP 4 273 215 A1**
(43) Date of publication of application: **08.11.2023**
(21) Application number: 21915894.6
(22) Date of filing: 31.12.2021
(51) Int. Cl.: C12M 1/34, C12M 3/00, C12N 5/09, C12N 5/071, G01N 33/50

(54) **SYSTEM FOR MEASURING METASTATIC POTENTIAL OF CANCER CELLS AND METHOD FOR MEASURING METASTATIC POTENTIAL OF CANCER CELLS BY USING SAME**

(30) Priority: 31.12.2020 KR 20200189281
(71) Applicant: Seoul National University Hospital, Seoul 03080 (KR)
(72) Inventor: CHO, Young-Jae, Seongnam-si, Gyeonggi-do 13599 (KR); EO, Eun Young, Yongin-si, Gyeonggi-do 16866 (KR); CHO, Sukki, Seongnam-si, Gyeonggi-do 13620 (KR)
(74) Representative: V.O.
(86) International application number: PCT/KR2021/020340
(87) International publication number: WO 2022/146097

(57) **Abstract**

The present specification relates to a system for measuring the metastatic potential of cancer cells and a method for measuring the metastatic potential of cancer cells by using same, the system comprising a microenvironment mimicking part and a measurement unit, in which the microenvironment mimicking part comprises: a primary tumor layer including cancer cells and vascular endothelial cells; and a metastasis layer including a gel. The system according to an aspect of the present invention has excellent effects such as being able to co-culture cancer cells and vascular endothelial cells, mimic the metastasis of cancer cells that is caused by a vascular layer formed by vascular endothelial cells during co-culture, easily observe and measure the metastatic potential of cancer cells by using a marker that is expressed specifically in cancer cells, and mimic the metastasis of cancer cells and measure the metastatic potential thereof even when patient-derived cancer cells as well as commercialized cancer cells are applied. Furthermore, the system can screen for a drug for suppressing cancer cell metastasis, and thus, by using same, there is an excellent effect of realizing personalized medicine based on actual clinical practice.

## Description

### Technical Field

The present specification is a system for measuring metastatic potential of cancer cells and a method for measuring the metastatic potential of cancer cells by using same, the system comprising a microenvironment mimicking unit and a measurement unit, in which the microenvironment mimicking unit comprises: a primary tumor layer including cancer cells and vascular endothelial cells; and a metastasis layer including a gel.

### [National Research and Development Project Supporting This Invention]

[Project Identification Number] 1711087541
[Project Number] 2017R1C1B1008061
[Name of Department] Ministry of Science and ICT
[Name of Project Management (Professional) Institution] National Research Foundation of Korea
[Research Business Name] Individual Basic Research (Ministry of Science and ICT) (R&D)
[Research Project Title] Exploratory Research on New Drug Candidate for Metastatic Lung Cancer Using Patient-Specific Lung Organ Chip
[Contribution Rate] 1 / 1
[Name of Project Performing Organization] Seoul National University Bundang Hospital
[Research Period] March 2, 2017 to February 29, 2020

### Background Art

Currently, blood tests, imaging tests, biopsies, genome tests, and the like are used as methods for diagnosing neoplastic diseases. Among the tests, whole-body PET (positron emission tomography) scan is used as a method of evaluating whether there is actual metastasis, but only presence of metastasis can be determined, and there is no method for evaluating how much metastatic potential the cancer cells identified in the primary tissue has.

Moreover, the clinical importance of lung cancer nationally and globally is unquestionable. In particular, the type of carcinoma in which targeted therapy and immunotherapy have been in the spotlight recently is lung cancer, which is a field in which so-called personalized medicine is actually becoming a reality.

Meanwhile, lung cancer still ranks first in the national cancer mortality rate. Unlike in the past, as early detection of lung cancer increased, the proportion of patients undergoing curative surgery increased, while patients who are diagnosed with stage 4 lung cancer due to metastasis to other organs at the time of diagnosis still account for equal to or more than 80% of the lung cancer patients and have a poor prognosis. In particular, it is known that metastasis to the brain, bone, liver, adrenal gland, and the like is common in lung cancer. Brain metastasis often causes disruption in daily life, and bone metastasis often causes pathological fractures and pain. Therefore, in both cases, metastasis has a more significant impact on deterioration in the quality of life than the lung cancer itself. Currently, there is no set principle or personalized treatment for metastatic lung cancer, and the need for a more accurate understanding of the mechanism of metastasis and an adjuvant or additional treatment suitable for the mechanism is clear.

It is known that the tumor microenvironment has a great influence on cancer metastatic cascade, and recently, a research team in Korea has published the results of preclinical animal experiment of a new antibody treatment in which the intratumoral delivery of existing anticancer drugs is increased through the normalization particularly of vascular tissues in the tumor microenvironment where metastasis occurs to reduce the size and also reduce metastasis. If there is a method to apply this to the clinical situation more quickly, the antibody treatment can be positioned as a breakthrough anticancer adjuvant treatment, and one of the best methods to bridge a gap between these preclinical experiments and clinical trials on actual patients is research using biomimetic organ chips.

The reason why a microengineered cancer metastasis model is needed although there are already existing experimental models for conducting research on cancer metastasis is that a biomimetic organ chip has its own advantages that go beyond existing 2D-based in vitro models and in vivo animal models.

Accordingly, the present inventors have conducted research on a biomimetic organ chip capable of measuring the metastatic potential of cancer cells beyond mimicking cancer metastasis, have conducted research to develop patient-specific organ chips based on actual clinical practice in addition to the use of already commercialized cell lines, and have completed the present invention capable of realizing personalized medicine.

### [Related Art]

### [Patent Literature]

1. JP 2009-027928A
2. KR 10-2016-0106105A
3. JP 1993-211893A

### Disclosure

### Technical Problem

The present inventors conducted research on a system capable of mimicking a tumor microenvironment by applying not only commercially available cancer cells but also patient-derived cancer cells and measuring the metastatic potentials of the applied cancer cells.

As a result of the research, it is confirmed that a system for measuring metastatic potential of cancer cells comprising a tumor microenvironment mimicking unit that includes a primary tumor layer including cancer cells and vascular endothelial cells; and a metastasis layer including a gel, and in which the cancer cells and the vascular endothelial cells are co-cultured, and a measurement unit that measures a metastatic potential of the cancer cells can measure metastatic potentials of both commercially available cancer cells and patient-derived cancer cells, to complete the present invention.

Therefore, according to an aspect, an object of the present invention is to provide a system for measuring a metastatic potential of cancer cells comprising a tumor microenvironment mimicking unit that includes a primary tumor layer including cancer cells and vascular endothelial cells; and a metastasis layer including a gel, and a measurement unit that measures a degree of migration of cancer cells from the primary tumor layer to the metastasis layer, in which the vascular endothelial cells in the primary tumor layer is directed toward the metastasis layer, and the cancer cells and the vascular endothelial cells are co-cultured.

According to another aspect, an object of the present invention is to provide a method for measuring a metastatic potential of cancer cells by using the system for measuring a metastatic potential of cancer cells.

According to another aspect, an object of the present invention is to provide a cancer cell metastasis suppressing drug screening system comprising a tumor microenvironment mimicking unit that includes a primary tumor layer including cancer cells and vascular endothelial cells; and a metastasis layer including a gel, and a measurement unit that measures a degree of migration of cancer cells from the primary tumor layer to the metastasis layer, in which the vascular endothelial cells in the primary tumor layer is directed toward the metastasis layer, the cancer cells and the vascular endothelial cells are co-cultured, and a cancer cell metastasis suppressing candidate is treated.

According to another aspect, an object of the present invention is to provide a cancer cell metastasis suppressing drug screening method using the cancer cell metastasis suppressing drug screening system.

### Solution to Problem

An aspect of the present invention provides a system for measuring a metastatic potential of cancer cells comprising a tumor microenvironment mimicking unit that includes a primary tumor layer including cancer cells and vascular endothelial cells; and a metastasis layer including a gel, and a measurement unit that measures a degree of migration of cancer cells from the primary tumor layer to the metastasis layer, in which the vascular endothelial cells in the primary tumor layer is directed toward the metastasis layer, and the cancer cells and the vascular endothelial cells are co-cultured.

Another aspect of the present invention provides a method for measuring a metastatic potential of cancer cells including culturing cancer cells in the primary tumor layer of the system for measuring a metastatic potential of cancer cells; and measuring a metastatic potential of cancer cells after the culturing of cancer cells.

Another aspect of the present invention provides a cancer cell metastasis suppressing drug screening system comprising a tumor microenvironment mimicking unit that includes a primary tumor layer including cancer cells and vascular endothelial cells; and a metastasis layer including a gel, and a measurement unit that measures a degree of migration of cancer cells from the primary tumor layer to the metastasis layer, in which the vascular endothelial cells in the primary tumor layer is directed toward the metastasis layer, the cancer cells and the vascular endothelial cells are co-cultured, and a cancer cell metastasis suppressing candidate is treated.

Another aspect of the present invention provides a cancer cell metastasis suppressing drug screening method including treating the cancer cell metastasis suppressing candidate to the primary tumor layer of the cancer cell metastasis suppressing drug screening system; and measuring a metastatic potential of the cancer cells after the treatment of the candidate.

### Advantageous Effects of the Invention

A system according to an aspect of the present invention has excellent effects of being able to co-culture cancer cells and vascular endothelial cells, mimic the metastasis of cancer cells passing through a blood vessel structure formed by the vascular endothelial cells during co-culture, easily observe and measure the metastatic potential of cancer cells by using a marker that is expressed specifically in cancer cells, and mimic the metastasis of cancer cells and measure the metastatic potential even when patient-derived cancels as well as commercialized cancer cells are applied. Further, the system according to an aspect of the present invention can screen a drug for suppressing cancer cell metastasis, and thus, by using same, there is an excellent effect of realizing personalized medicine based on actual clinical practice.

### Brief Description of Drawings

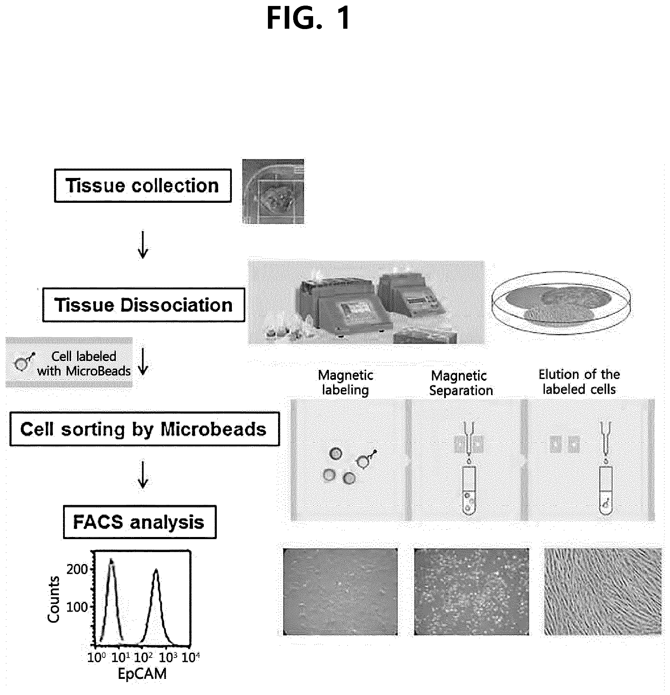

FIG. 1 is a diagram illustrating a cell separation process according to an aspect of the present invention.

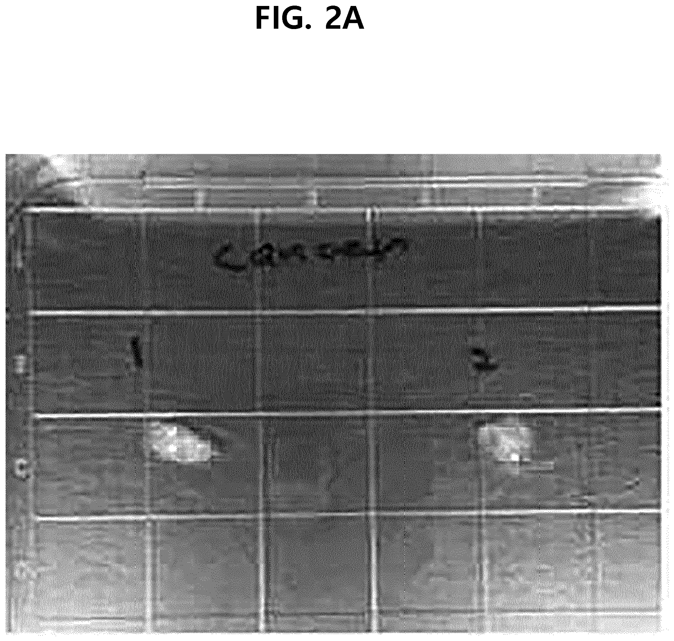

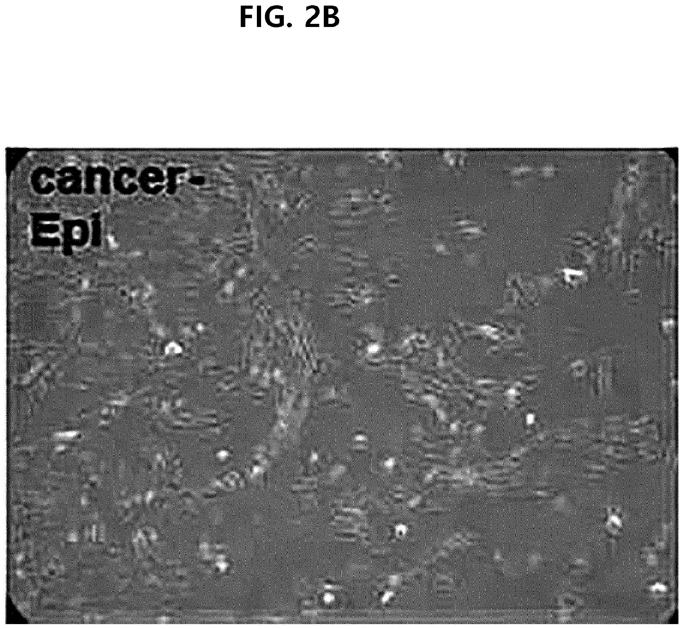

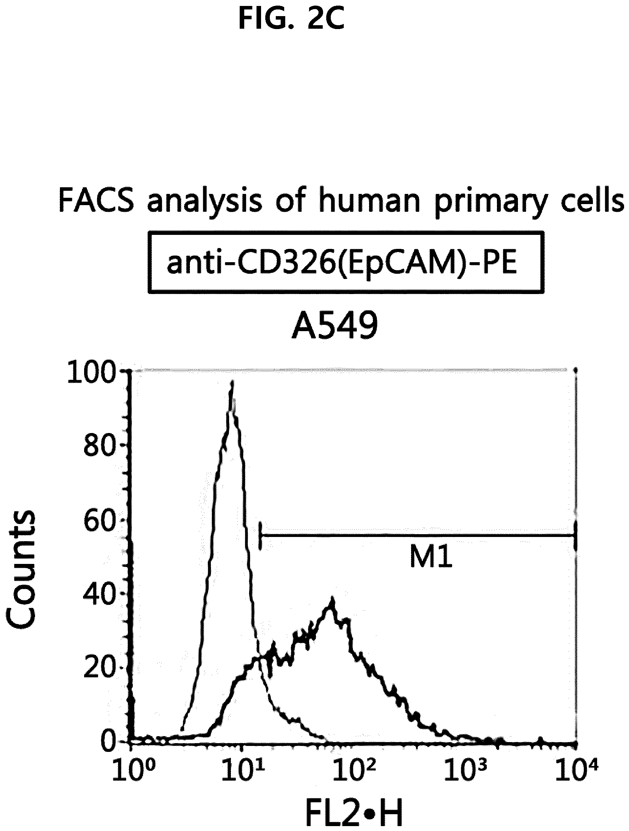

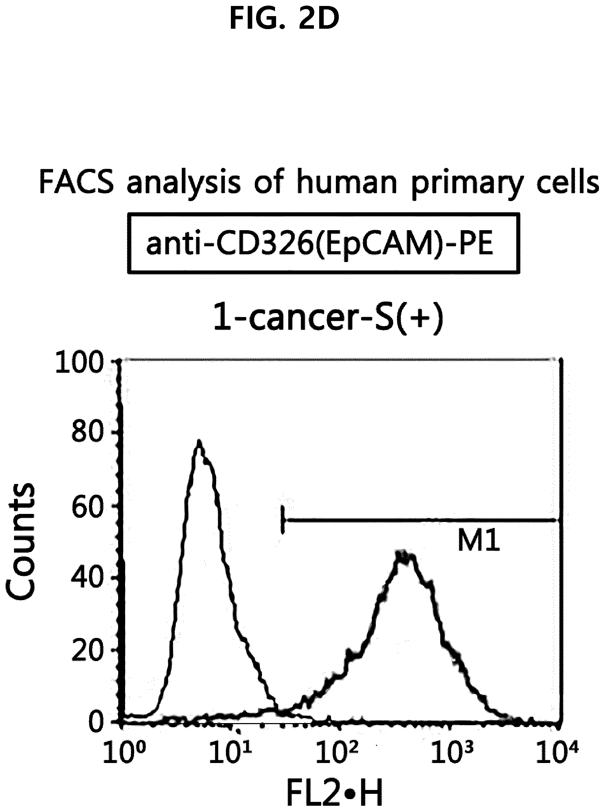

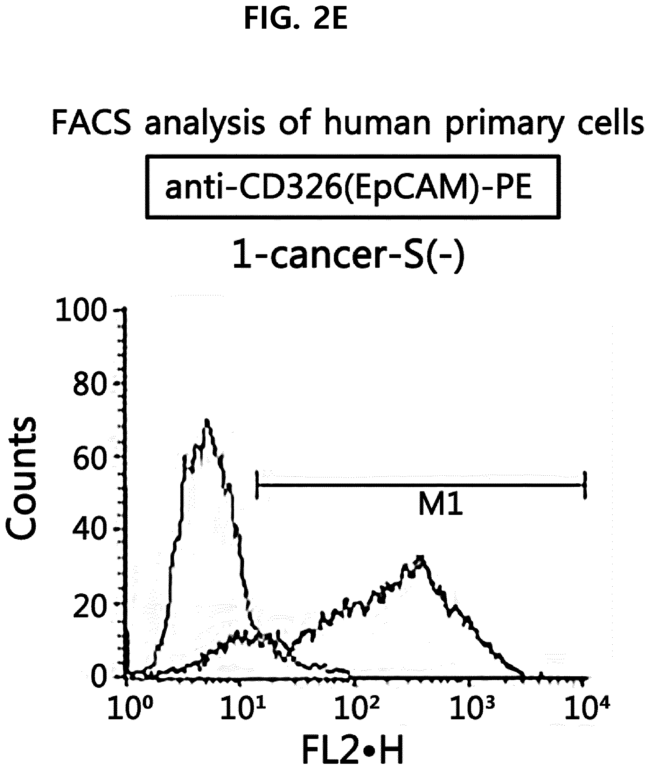

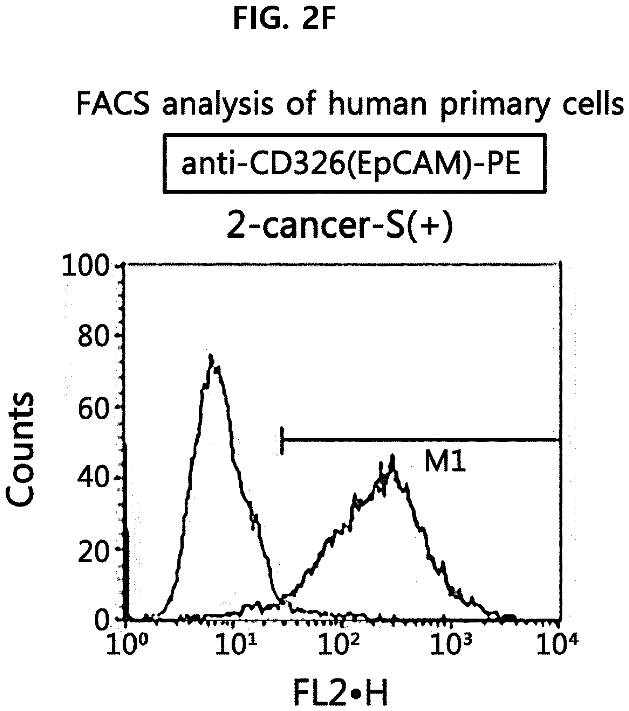

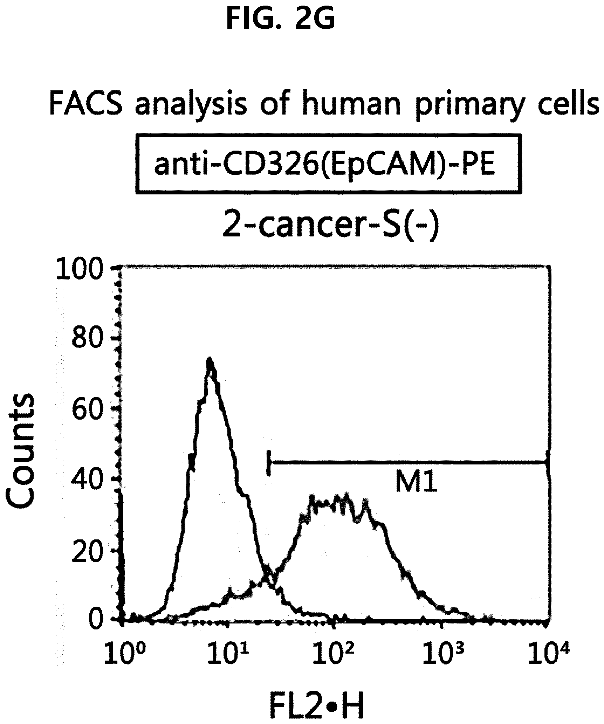

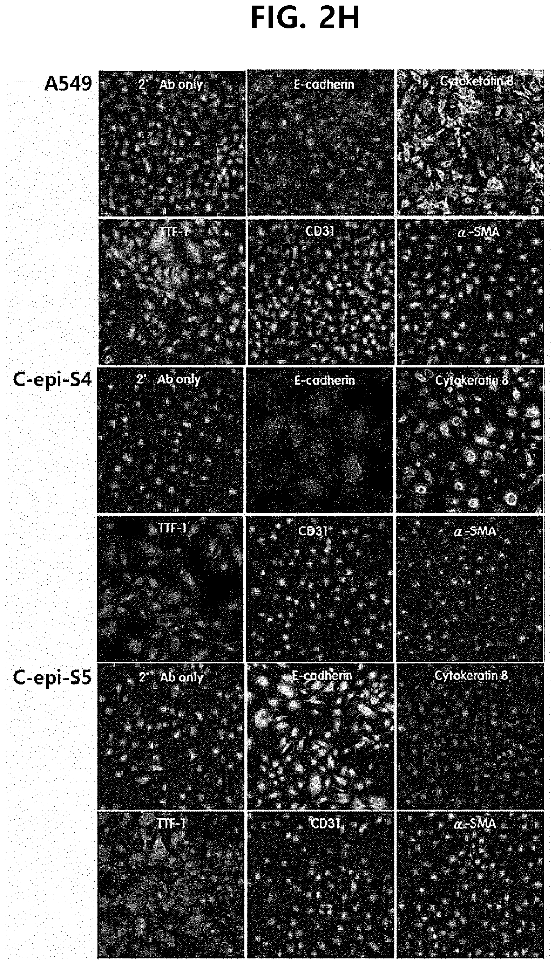

FIG. 2A is a diagram illustrating a photograph of tumor tissues (cancer tissue Nos. 1 and 2) separated from a patient according to a method according to an aspect of the present invention. FIG. 2B is a photograph showing epithelial cells (cancer-Epi) of the separated tumor tissue. FIGS. 2C to 2G are diagrams illustrating FACS analysis results of lung cancer cells (1-cancer and 2-cancer) obtained from tumor tissues separated from patients and commercially available lung cancer cells (A549). FIG. 2h is photographs showing expressions of specific markers in lung cancer cells (C-epi-S4 and C-epi-S5) obtained from the patient and commercially available lung cancer cells (A549).

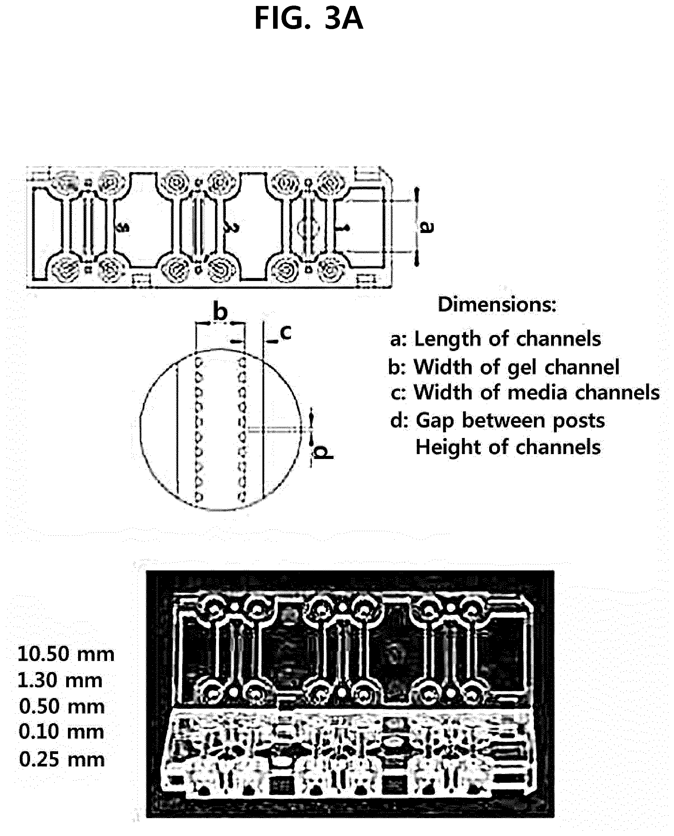

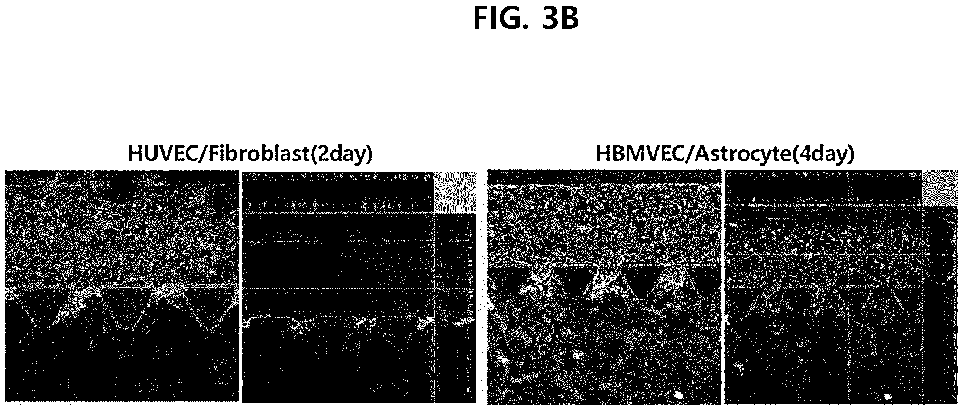

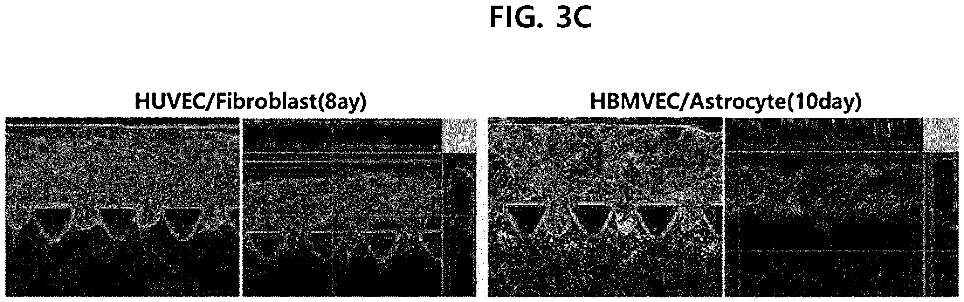

FIG. 3A is a schematic diagram and a photograph showing an AIM chip according to an aspect of the present invention. FIG. 3B is photographs showing structures of lumens of vascular endothelial cells in a lung mimicking model (HUVEC/Fibroblast) and a BBB mimicking model (HBMVEC/Astrocyte), respectively, obtained by seeding vascular endothelial cells (HUVEC and HBMVEC), and fibroblasts or astrocytes to the AIM chips by the method according to an aspect of the present invention (the left illustrates a case where vascular endothelial cells (HUVEC) and fibroblasts were co-cultured for two days, and the right illustrates a case where vascular endothelial cells (HBMVEC) and astrocytes were co-cultured for four days). FIG. 3C is a photographs showing blood vessel formation of vascular endothelial cells when being cultured for eight days and ten days, respectively, in each co-culture system.

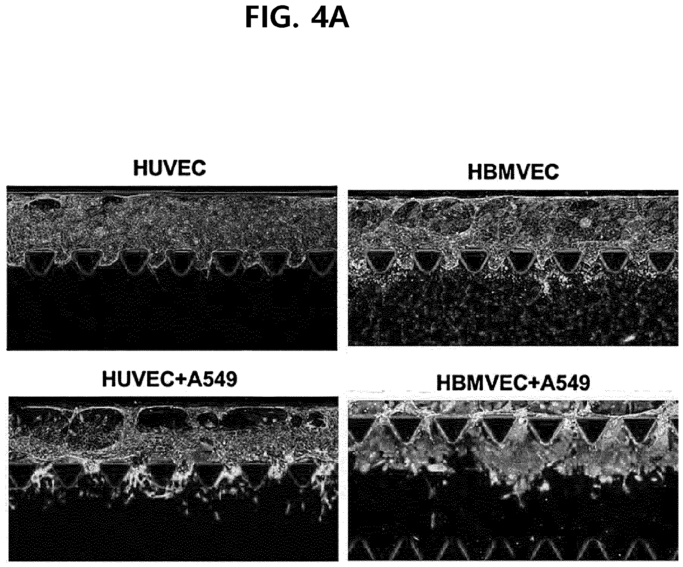

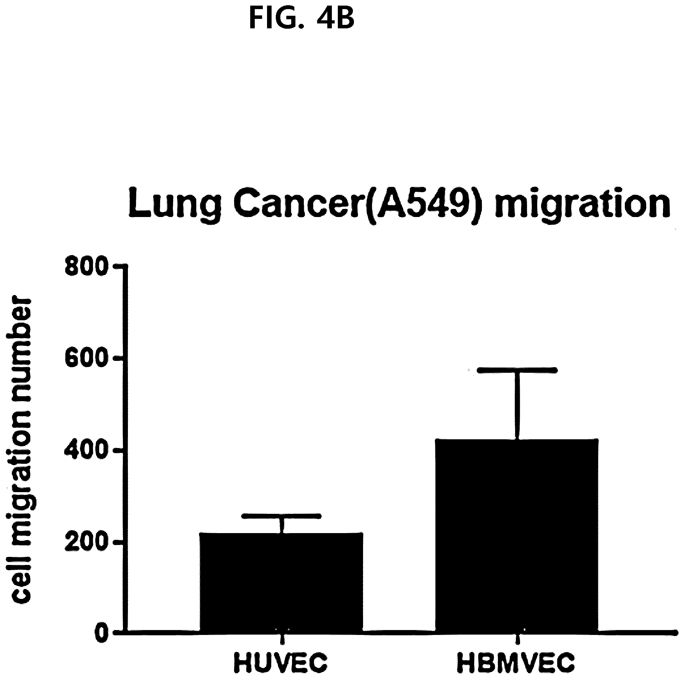

FIG. 4A is photographs showing the metastatic potentials of the lung cancer cells (A549) in each of the lung mimicking model and the BBB mimicking model in the system according to an aspect of the present invention, and FIG. 4B is a graph obtained by comparing the metastatic potentials of the lung cancer cells in the respective models by measuring and quantifying degrees of migration of the lung cancer cells.

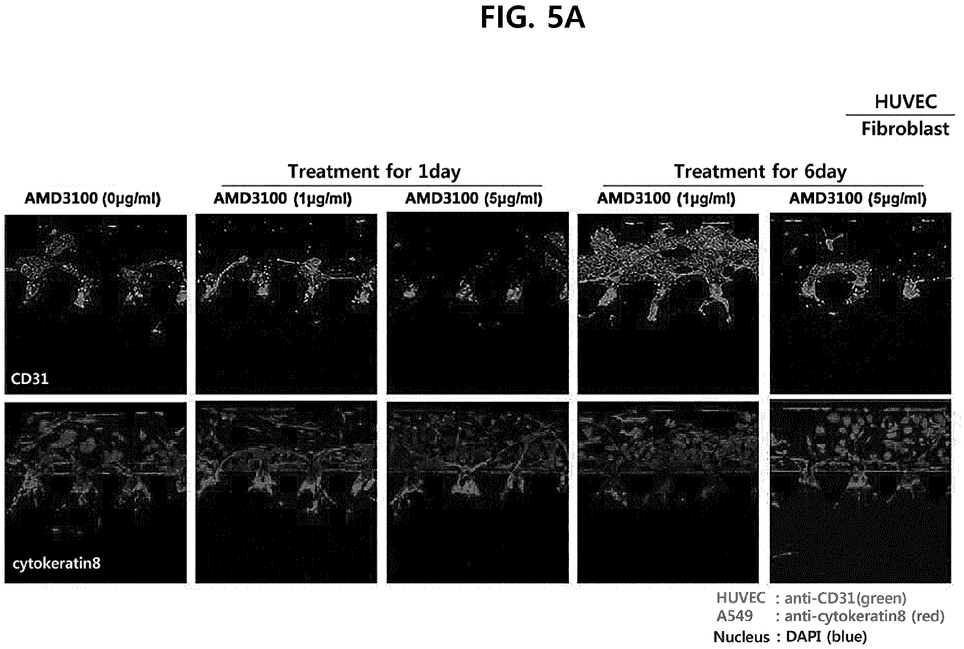

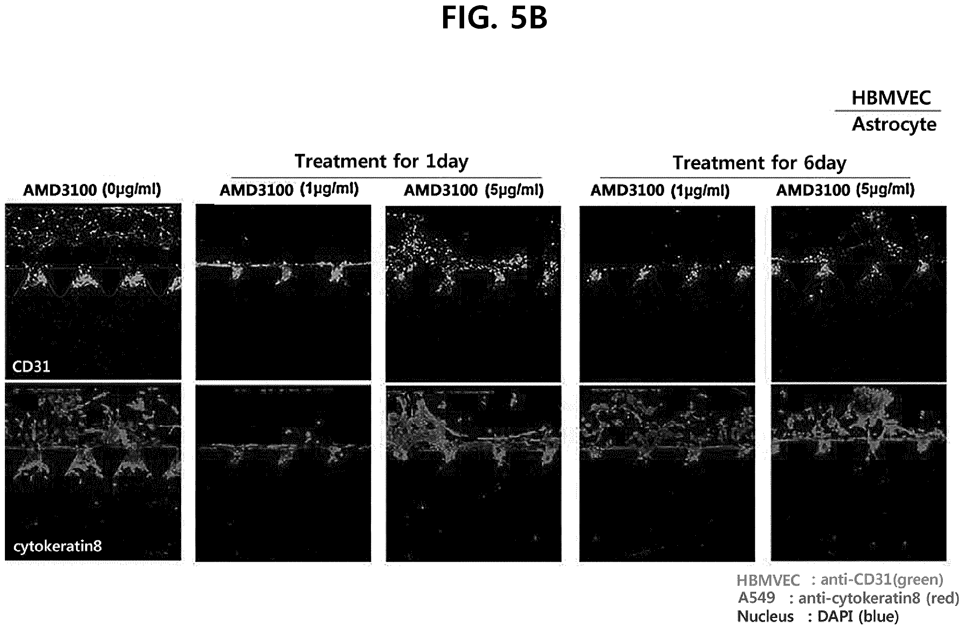

FIGS. 5A and 5B are photographs obtained by photographing metastasis suppressing effects when AMD3100, which is known to suppress metastasis of lung cancer cells to the brain, is treated to each of the lung mimicking model (FIG. 5A) and the BBB mimicking model (FIG. 5B) in the system according to an aspect of the present invention.

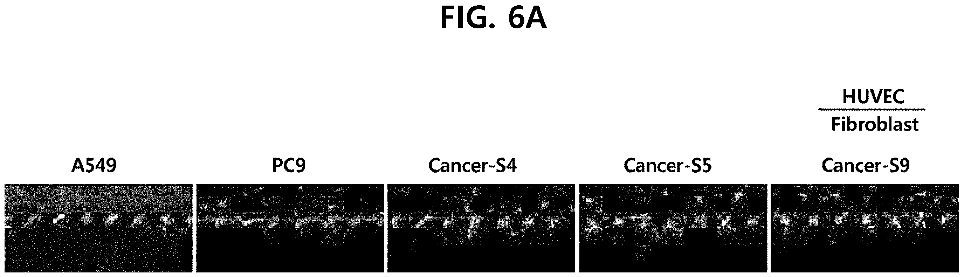

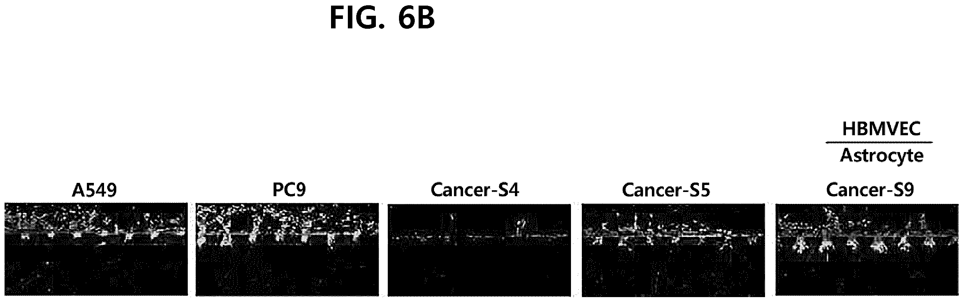

FIGS. 6A and 6B are photographs obtained by photographing metastatic potentials of the lung cancer cells in each of the lung mimicking model (FIG. 6A) and the BBB mimicking model (FIG. 6B) in systems manufactured by using lung cancer cells separated from patients (Cancer-S4, S5, and S9) and commercially available lung cancer cells (A549 and PC9) according to an aspect of the present invention.

### Mode for Invention

Hereinafter, the present invention is specifically described.

According to an aspect of the present invention, "metastasis" refers to the spread of cancer cells from an initial site of origin (that is, a primary tumor site) to another site (that is, a metastatic site), and "measuring metastatic potential of cancer cells" refers to measuring a metastatic ability or a metastatic potential of cancer cells to a metastasis suspected tissue.

According to an aspect of the present invention, terms such as "unit", "module", "device", and "system" may refer to not only hardware but also a combination of software driven by the corresponding hardware, if necessary. For example, the hardware may be a data processing device including a CPU or other processor. Also, the software driven by the hardware may be a program such as a running process, an object, an executable file, a thread of execution, or a calculation program.

According to an aspect, the present invention provides a system for measuring the metastatic potential of cancer cells, the system comprising a tumor microenvironment mimicking unit and a measurement unit, in which the tumor microenvironment mimicking unit comprises: a primary tumor layer including cancer cells and vascular endothelial cells; and a metastasis layer including a gel, the vascular endothelial cells in the primary tumor layer is directed toward the metastasis layer, the cancer cells and the vascular endothelial cells are co-cultured, and the measurement unit measures a degree of migration of cancer cells from the primary tumor layer to the metastasis layer.

According to an aspect of the present invention, the system for measuring the metastatic potential of cancer cells may include a tumor microenvironment mimicking unit and a measurement unit.

According to an aspect of the present invention, the tumor microenvironment mimicking unit may include the primary tumor layer; and the metastasis layer.

According to an aspect of the present invention, the tumor microenvironment mimicking unit may mimic a primary tumor site or a metastatic site, and specifically mimic one or more tissues selected from the group consisting of the lung, the brain, the bladder, the bone, the bone marrow, the breast, the cervix, the colon, the endometrium, the esophagus, the intestine, the kidney, the liver, the oral cavity, the muscle, the ovary, the skin, the pancreas, the prostate, the skin, the stomach, the testis, the thyroid, and the uterus, but the present invention is not limited thereto. In addition, the tumor microenvironment mimicking unit may mimic any hyperproliferative tissue including a blood vessel structure, and specifically, the blood vessel structure may include endothelial cells, smooth muscle cells, pericytes, scars, fibrotic tissues, surgery adhesions, or hyperproliferative bone lesions.

According to an aspect of the present invention, the primary tumor layer may include cancer cells, the cancer cells may be one or more cancer cells selected from the group consisting of lung cancer, breast cancer, melanoma, small cell lung cancer, non-small cell lung cancer, glioma, liver cancer, thyroid tumor, stomach cancer, prostate cancer, ovarian cancer, bladder cancer, colorectal cancer, prostate cancer, glioblastoma, endometrial cancer, kidney cancer, colon cancer, pancreatic cancer, esophageal carcinoma, head and neck cancer, mesothelioma, sarcoma, cholangiocarcinoma, small intestine adenocarcinoma, pediatric malignancy, and epidermal cancer, specifically may be one or more cancer cells selected from the group consisting of lung cancer cells and breast cancer cells, and more specifically lung cancer cells, but the present invention is not limited thereto.

According to an aspect of the present invention, the cancer cells may be treated when blood vessels are formed in the tumor microenvironment mimicking unit. The metastatic potential of cancer cells can be measured by treating the cancer cells after blood vessels are formed in the tumor microenvironment mimicking unit and measuring a degree in which the cancer cells of the primary tumor layer migrates to the metastasis layer.

According to an aspect of the present invention, the primary tumor layer may include vascular endothelial cells, and types of the vascular endothelial cells may vary depending on a mimicking target of the tumor microenvironment mimicking unit. For example, when the mimicking target of the tumor microenvironment mimicking unit is a lung microenvironment, the vascular endothelial cells may include lung microvascular endothelial cells or umbilical vein endothelial cells. When the mimicking target of the tumor microenvironment mimicking unit is brain microenvironment, the vascular endothelial cells may include brain microvascular endothelial cells.

According to an aspect of the present invention, the vascular endothelial cells in the primary tumor layer may be directed toward the metastasis layer. Since the vascular endothelial cells are attached in the direction of the metastasis layer, a vascular layer may be formed starting from the metastasis layer, and cancer cells of the primary tumor layer may migrate to the metastasis layer through the formed vascular layer.

According to an aspect of the present invention, the metastasis layer may include a gel, specifically, the gel may be an extracellular matrix (ECM), and more specifically may be one or more selected from the group consisting of collagen, fibronectin, and gelatin. However, the type of the gel is not limited as long as cells present in the system according to an aspect of the present invention, specifically, vascular endothelial cells can be attached and cultured in the gel, and the gel enables migration of cancer cells.

According to an aspect of the present invention, the metastasis layer may further include cells to form a lumen of a blood vessel according to a mimicking target of the tumor microenvironment mimicking unit. Specifically, when the mimicking target of the tumor microenvironment mimicking unit is lung microenvironment, the metastasis layer may further include fibroblasts. On the other hand, when the mimicking target of the tumor microenvironment mimicking unit is brain microenvironment, the metastasis layer may further include astrocytes. When fibroblasts are additionally seeded and cultured in a metastasis layer of the tumor microenvironment mimicking unit desired to mimic the lung microenvironment, the fibroblasts are in a space adjacent to the vascular endothelial cells, and thus there is an effect of better forming the lumen of the blood vessel.

According to an aspect of the present invention, the cancer cells and the vascular endothelial cells may be co-cultured. Conditions for culturing the cancer cells or the vascular endothelial cells may vary depending on the type of cancer cells or the type of microenvironment to be mimicked by the tumor microenvironment mimicking unit.

According to an aspect of the present invention, the measurement unit may measure a degree of migration of cancer cells from the primary tumor layer to the metastasis layer. Specifically, the degree of migration of cancer cells may be measured by measuring the expression level of a cancer cell-specific marker. More specifically, the epithelial cell-specific marker may be E-cadherin or cytokeratin 8 (CCK8), the fibroblast-specific marker may be α-smooth muscle actin (α-SMA), and the vascular endothelial cell-specific marker may be cluster of differentiation 31 (CD31). However, the specific marker may vary depending on the type of cancer cell. Further, specifically, the measurement unit may quantify and measure the expression level of the cancer cell-specific marker, and more specifically, the expression of the specific marker may be the expression of a specific fluorescent marker.

According to an aspect of the present invention, the cancer cells or the vascular endothelial cells may be separated from a human tissue. In addition, the cells, specifically, fibroblasts or astrocytes that are added according to the mimicking target of the tumor microenvironment mimicking unit may be separated from a human tissue.

According to another aspect, the present invention provides a method for measuring the metastatic potential of cancer cells including: culturing cancer cells in the primary tumor layer of the system for measuring the metastatic potential of cancer cells; and measuring a metastatic potential of cancer cells after the culturing of the cancer cells. The description of the system for measuring the metastatic potential of cancer cells, the primary tumor layer, the cancer cells, the measuring of metastatic potential, and the like are as described above.

According to an aspect of the present invention, the culturing of cancer cells may be seeding and culturing after a blood vessel is formed in the tumor microenvironment mimicking unit of the system.

According to an aspect of the present invention, the metastasis layer of the system may additionally culture cells for forming a vascular network depending on the mimicking target of the tumor microenvironment mimicking unit. Specifically, when the mimicking target of the tumor microenvironment mimicking unit is lung microenvironment, the metastasis layer may additionally culture fibroblasts. On the other hand, when the mimicking target of the tumor microenvironment mimicking unit is brain microenvironment, the metastasis layer can further culture astrocytes.

According to an aspect of the present invention, in the method for measuring the metastatic potential of cancer cells, the cancer cells or the vascular endothelial cells may be separated from a patient, and cells additionally cultured according to the mimicking target of the tumor microenvironment mimicking unit may be also separated from the patient. According to an embodiment of the present invention, lung cancer cells separated from lung cancer patients were cultured in the system according to an aspect of the present invention and the metastatic potential of cancer cells thereof was measured, so that it was found that the patient-specific metastatic potential of cancer cells was able to be measured, and thus personalized medicine was able to be realized (Example 3 and Experimental Example 3).

According to an aspect of the present invention, the measuring of the metastatic potential of cancer cells may be to measure a degree of migration of cancer cells from the primary tumor layer to the metastasis layer, and the degree of migration of cancer cells is measured as described above.

Also, according to another aspect, the present invention is a cancer cell metastasis suppressing drug screening system, the system comprising a tumor microenvironment mimicking unit; and a measurement unit, in which the tumor microenvironment mimicking unit comprises a primary tumor layer including cancer cells and vascular endothelial cells; and a metastasis layer including a gel, the vascular endothelial cells in the primary tumor layer is directed toward the metastasis layer, the cancer cells and the vascular endothelial cells are co-cultured, the measurement unit measures a degree of migration of the cancer cells from the primary tumor layer to the metastasis layer, and the system is treated a cancer cell metastasis suppressing candidate. The description on the cancer cell, the tumor microenvironment mimicking unit, the measurement unit, the vascular endothelial cells, the primary tumor layer, the gel, the metastasis layer, the co-culturing, the measurement unit, and the measuring of a degree of migration of cancer cells is as described above.

According to an aspect of the present invention, the measurement unit may measure the degree of migration of cancer cells from the primary tumor layer to the metastasis layer in the system before and after treatment of the cancer cell metastasis suppressing candidate.

Also, according to an aspect of the present invention, when a degree of migration of cancer cells after the treatment of the cancer cell metastasis suppressing candidate is reduced compared to that before the treatment with the candidate, the cancer cell metastasis suppressing drug screening system may determine the candidate as a cancer cell metastasis suppressing drug.

According to an embodiment of the present invention, when AMD3100, which is known as a cancer cell metastasis suppressing drug, is treated to the system according to an aspect of the present invention, the metastasis suppressing effects of the drug in each of the lung mimicking model and the BBB mimicking model were exhibited differently, and the effect was different depending on the dosage and administration period. Therefore, it was found that drugs capable of suppressing metastasis of cancer cells were able to be screened by using the cancer cell metastasis suppressing drug screening system according to an aspect of the present invention (Experimental Example 2).

Also, according to another aspect, the present invention provides a cancer cell metastasis suppressing drug screening method including: treating the cancer cell metastasis suppressing candidate to the primary tumor layer of the cancer cell metastasis suppressing drug screening system; and measuring a metastatic potential of cancer cells after the treatment of the candidate. The description of the cancer cell metastasis suppressing drug screening system, the primary tumor layer, the cancer cells, the measuring of metastatic potential of cancer cells, and the like are as described above.

According to an aspect of the present invention, the screening method may further include: measuring a metastatic potential of cancer cells before the treatment of the candidate; and determining the candidate as a cancer cell metastasis suppressing drug when a degree of migration of cancer cells after the treatment of the cancer cell metastasis suppressing candidate is reduced compared to that before the treatment of the candidate.

Hereinafter, the configurations and effects of the present invention are more specifically described with reference to Examples and Experimental Examples. However, the following Examples and Experimental Examples are provided only for illustrative purposes for better understanding of the present invention, and the category and the scope of the present invention are not limited thereto.

### [Example 1] Lung Cancer Cell Separation from Patient Tissue

In order to manufacture a system including cells separated from a patient, lung cancer cells were first separated and cultured in the method as below.

First, samples in Table 1 obtained from tumor tissues among resected lung tissues of a lung cancer patient who underwent surgery were placed in a tissue storage buffer (Mitenyi Biotec, MACS tissue storage solution) and stored at 4°C for up to one day.

**[Table 1]**

| Tissue Sample | Diagnosis |
|---|---|
| C-S4 | Adenocarcinoma |
| C-S5 | Adenocarcinoma |
| C-S9 | Large Cell Neuroendocrine Carcinoma (60%) Mixed with Small Cell Carcinoma (40%) |

Then, 4.7 ml of a RPMI culture medium (Welgene, RPMI 1640) that was a serum-free culture medium was dispensed into each C-tube (Miltenyi biotec) for tissue separation, and enzymes of an enzyme kit (Miltenyi biotec, Tumor dissociation kit, human) were added to each of the C-tubes according to the instructions. Residual blood of lung cancer tissue was removed by washing with PBS (FIG. 2A), and approximately 0.5 g of the tissues were placed in the C-tube with enzymes. After that, scissors were inserted into the C-tube to cut the tissue into small pieces to facilitate dissociation, and then the tube was inserted into a dissociation device (Miltenyi biotec, gentleMACStm Octo Dissociator with heaters) and operated by selecting a program suitable for the dissociation kit (37°C_h_TDK_2). A sieve was prepared in a 50-ml tube and soaked with about 2 ml of the serum-free RPMI culture medium, and after the end of the program, lung tissue samples included in each C-tube were filtered through the sieve. Thereafter, the supernatant was removed by centrifugation at 300 × g for 10 minutes, 1 ml of a red blood cell lysis buffer (LONZA) was added, and the mixture was left for two minutes at room temperature and then resuspended in 5 ml of the serum-free RPMI medium. Then, the supernatant was removed by centrifugation at 300 × g for 10 minutes, and the cells were counted after resuspending with 5 ml or 10 ml of lung cancer cell culture medium (LONZA, SAGM), then placed in a T-175 flask containing the culture medium, and cultured to dissociate the lung tissue of the patient. The cultured cells were observed under an optical microscope and photographed, and the results are as in FIG. 2B.

Then, after the cultured cells were counted and approximately 1 × 107 cells were prepared, the supernatant was removed by centrifugation at 300 × g at room temperature for 10 minutes, and the cells were resuspended in 3 ml of a PEB solution (autoMACS rinsing solution #130-091-222 and MACS BSA stock solution #130-091-376 were mixed in a ratio of 20:1). Then, in order to increase the purity of the separated cells, 100 ul of an FcR blocking solution (Miltenyi biotec, #130-059-901) and 100 ul of a CD326 microbead (Miltenyi biotec, #130-061-901) were respectively put into the epithelial cell suspension and mixed, and the treatment was performed for 30 minutes in a refrigerator. Then, 2 ml of the PEB solution was added and centrifuged at 300 × g for 10 minutes, and the supernatant was removed and resuspended in 2 ml of the PEB solution.

In order to separate the cells by using the magnetism of the microbeads in the suspension (magnetic separation), an LS column was inserted into the magnetic body of a cell separator (Miltenyi biotec, MACStm separator), 3 ml of the PEB solution was put down into the column, and then the prepared cells were taken down. After washing the column with 3 ml of PEB solution a total of three times, the column was removed from the separator and placed in a new tube, 5 ml of PEB solution was added, and a plunger was pushed down until bubbles appeared. The tube containing the lung epithelial cells (labeled cells) to which the CD326 microbeads that came out through the column were bound and labeled was centrifuged at 300 × g for 10 minutes, the supernatant was removed and resuspended in 10 ml of a cell culture agent, and the epithelial cells were counted and put into the T-175 flask containing the culture medium and cultured.

Thereafter, fluorescence activated cell sorter (FACS) analysis was performed to compare the separated lung cancer cells with an existing commercially available lung cancer cell line (A549).

Specifically, cells were counted and prepared to be approximately 1 × 106, centrifuged at 300 × g for 10 minutes, the supernatant was removed, cells that were not treated with the antibody were resuspended in 1 ml of the PEB solution, and cells treated with the antibody were resuspended in 98 ul of the PEB solution. After adding 2 ul of the CD326-PE (Miltenyi biotec, CD326 (EpCAM)-PE #130-111-116) antibody to the cells resuspended in 98 ul, the cells were treated for 10 minutes in a refrigerator. Thereafter, 2 ml of the PEB solution was added, centrifuged at 300 × g for 10 minutes to remove the supernatant, and resuspended with 1 ml of the PEB solution. The prepared cells were analyzed by using FACS equipment (FLOW CYTOMETRY ANALYZER MODUAL) (Becton & Dickinson, FACS CALIBER), and the results are as shown in FIGS. 2C to 2G.

In addition, the lung cancer epithelial cells separated from the patient and the commercially available lung cancer cell line (A549) were compared by using a confocal microscope (CARL ZEISS, LSM710). The lung cancer cells separated according to the above and the commercially available lung cancer cell line (A549) was reacted, as a primary antibody, with a mouse monoclonal antibody anti-E-cadherin (mouse mAb anti-E cadherin) (Abcam, ab 1416) that was an epithelial cell marker at 1:100, was reacted with a mouse monoclonal antibody anti-cytokeratin 8 (mouse mAb anti-cytokeratin8) (Santa cruz, sc-73480) that was another epithelial cell marker at 1:100, and was reacted with donkey anti-mouse IgG-Alexa 488 (anti-mouse IgG-Alexa 488) (Abcam, ab150105) at a ratio of 1:200 as a secondary antibody, the resultants were photographed with a confocal microscope (Carl Zeiss, LSM710), and the results are shown in FIG. 2H.

In addition, the expression of TTF-1 which is checked when diagnosing lung cancer cells was reacted with the mouse monoclonal antibody anti-TTF-1 (mouse anti-TTF-1) (Santa cruz, sc-53136), that was the primary antibody, at 1:50, was reacted with donkey anti-mouse IgG-Alexa 488 (anti-mouse IgG-Alexa 488) (Abcam, ab 150105) as a secondary antibody at 1:200, and photographed with the confocal microscope to confirm the expression. Not only the separated lung cancer cells but also the used lung cancer cell line (A549) expressed TTF-1, and thus it was confirmed that the separated cells were cancer cells.

In addition, FIG. 2H shows that α-smooth muscle actin (α-SMA), which is a marker of fibroblasts, and cluster of differentiation 31 (CD31), which is a marker of vascular endothelial cells, were not expressed.

### [Example 2] Optimized System for Measuring Metastatic Potential of Cancer Cells Manufactured by Using Commercially Available Lung Cancer Cells

In order to construct a model for more accurately confirming the metastasis patterns of lung cancer, experiments were performed in the method as below. At this time, the chip used was the AIM chip illustrated in FIG. 3A, and a lung mimicking model for mimicking the environment of the lung and a BBB mimicking model for mimicking the BBB (blood brain barrier) were prepared, respectively.

First, in order to manufacture a lung mimicking model, 2 × 106/ml of fibroblasts separated from the patient of Example 1 were mixed with 2.5 mg/ml of fibronogen (Sigma, F8630) and filled to a gel channel in the middle of the AIM chip. Then, gelation was allowed at room temperature for 15 minutes. Then, after coating with collagen (0.03 mg/ml, 1 VL content) (sigma, collagen type I solution) and fibronectin (0.001%, 5 mg) (Sigma, F0895) for two hours at 37°C, a total of 20 µl of 4 × 106/ml of HUVEC (LONZA, HUVEC-Umbil vein, Pooled cells) as vascular endothelial cells was added into one of the cell culture channels, the chip was raised at 90°, and the cells were cultured for one to two hours so as to be adhered in a direction of the gel layer formed in the middle. Then, put the chip down to its original state, replace the medium, and when the vascular endothelial cells form one blood vessel layer starting from the gel layer in the middle, a total of 20 µl of 1X106/ml lung cancer cells (A549) were put on the same layer, the chip was raised at 90° and cultured overnight so that the chip was attached in the direction of the gel layer formed in the middle. Then, the chip was put down again, the medium was replaced, and cultured for 5 days, and it was confirmed whether the lung cancer cells migrated to the middle gel layer. After that, the chip was put down to the original state again, the medium was replaced, the cells were cultured for five days, and whether the lung cancer cells migrated to the middle gel layer was checked.

In addition, the BBB mimicking model was manufactured in the same method of manufacturing the lung mimicking model, but astrocytes (Human Astrocytes) (ScienCell, #1800) were used instead of fibroblasts, human brain microvascular endothelial cells (HBMVEC) (Cell systems, ACBRI 376) were used instead of HUVECs as vascular endothelial cells, and the total culture periods were set to be different due to the difference in cell proliferation rates between the lung mimicking model and the BBB mimicking model.

In order to confirm each cell, an antibody of a specific marker of each cell was reacted and identified. In the experimental method, as described in Example 1 above, blood vessel cells were treated with CD31, lung cancer cells were treated with cytokeratin 8, and the cells were photographed by using a confocal microscope, Carl Zeiss, LSM710.

The structure of lumens formed in the lung mimicking model and the BBB mimicking model is shown in FIG. 3B. As shown in FIG. 3B, it was checked that the structure of lumens of blood vessels was formed when cultured for two days in the lung mimicking model and four days in the BBB mimicking model. As shown in FIG. 3B, it was checked that as the culture period became longer, the structure of lumens of the initially formed blood vessels became more distinct.

### [Experimental Example 1] Confirmation of Difference in Metastatic Potentials of Lung Cancer Cells Between Lung Mimicking Model and BBB Mimicking Model

The metastatic potentials of the lung cancer cells in the lung mimicking model and the BBB mimicking model manufactured in Example 2 were measured and compared, and the results are shown in FIGS. 4A and 4B.

As illustrated in FIG. 4A, it was confirmed that commercially available lung cancer cells both in the lung mimicking model and the BBB mimicking model metastasized to the metastasis layer, and it was confirmed that even the same lung cancer cells showed different metastasis patterns depending on the environment of the mimicking model.

In order to quantitatively analyze the metastasis patterns of the lung cancer cells, analysis was performed using the IMARIS program (Bitplane, IMARIS FL). The results photographed by using the confocal microscope were applied to the software IMARIS to analyze the images. In the AIM structure of the chip used, the number of lung cancer cells migrated from the gel layer to the metastasis layer in the middle in blood vessel cells with respect to the port of the middle channel was objectively checked through the graph of FIG. 4B. Here, it was checked that the lung cancer cells (A549) showed more metastasis in the BBB mimicking model than in the lung mimicking model.

Through this, the system according to an aspect of the present invention can mimic various tissues, for example, the lung and the BBB, and induce or mimic metastasis of cancer cells in the microenvironment of each tissue to measure the metastatic potential of cancer cells in each tissue. Particularly, it was found that the metastatic potential of cancer cells can be effectively measured by easily checking with the naked eye the expression of a fluorescent marker specific to cancer cells and quantifying the expression.

### [Experimental Example 2] Screening of Metastasis Suppressing Drug of Lung Cancer Cells by Using System for Measuring Metastatic Potential of Cancer Cells

Through Experimental Example 1, it was confirmed that the metastasis potential of cancer cells can be evaluated by using the system according to an aspect of the present invention. The following experiments were performed by using the system for measuring a metastatic potential of cancer cells manufactured in Experimental Example 1 in order to find whether the metastasis suppressing drug of cancer cells can be screened by using the system. At this time, drug screening was performed with an emphasis on extravasation, a process in which cancer cells pass through cell walls of blood vessels during metastasis to other tissues. AMD3100 (octahydrochloride) (Sigma, A5602) used in this experiment is a strong CXCR4 antagonist and is a drug known to suppress metastasis of lung cancer cells to the brain by interfering with the SDF-1/CXCR4 axis to protect the BBB.

Specifically, when the lung mimicking model and the BBB mimicking model of Experimental Example 1 were made, and when the lung cancer cells were treated to a vascular cell layer, 1 µg/ml and 5 µg/ml of the AMD3 100 were mixed with the culture medium and treated for one day or six days, and the degree of metastasis of lung cancer cells was photographed by using a confocal microscope (Carl Zeiss, LSM710).

As illustrated in FIG. 5A, in the lung mimicking model, the metastasis of lung cancer cells was generally reduced in the treatment group compared to the group to which AMD3100 is not treated, but the group to which 1 µg/ml was treated for six days exhibited the best metastasis suppression.

Similarly, as shown in FIG. 5B, in the BBB mimicking model, metastasis of lung cancer cells was reduced more in the group to which AMD3100 was treated, and the group to which 1 µg/ml was treated for one day exhibited the best metastasis suppression.

According to this, it was found that a drug capable of suppressing metastasis of cancer cells can be screened by using the system according to an aspect of the present invention.

### [Example 3] Optimized System for Measuring Metastatic Potential of Cancer Cells Manufactured by Using Lung Cancer Cells Separated from Patient

The lung mimicking model and the BBB mimicking model were manufactured, respectively in the same manner as in Example 2, but the lung cancer cells used at this time were three types of lung cancer cells (CancerS4, S5, and S9) separated from the patient in Example 1. In addition to A549, PC9 (Korea Atomic Energy Hospital) that was non-small cell lung carcinoma (NSCLC) with EGFR mutation were additionally used as the commercially available lung cancer cells.

### [Experimental Example 3] Personalized Lung System for Measuring Metastatic Potential of Cancer Cells

In order to find whether the measurement of the personalized cancer cell metastatic potential and the drug screening can be performed by using the system according to an aspect of the present invention, the experiment was performed in the same method as Experimental Example 1 by using the system for measuring a metastatic potential of cancer cells manufactured in Example 3, and the results are shown in FIGS. 6A and 6B.

As shown in FIG. 6A, in the lung mimicking model, all other lung cancer cells except for the PC9 cell line showed that metastasis was exhibited well.

Meanwhile, as shown in FIG. 6B, in the BBB mimicking model, it was checked that metastasis occurred in the model prepared by using other cells (Cancer-S5 and S9 which were patient-derived cells and commercially available A549 and PC9 cells) except for Cancer-S4 cells which were patient-derived cells.

From the above results, it was found that metastasis of lung cancer cells separated from patients occurred differently depending on each environment, and in particular, through a model manufactured by using Cancer-S5 and S9 which were patient-derived cells, the two types of lung cancer cells were likely to metastasize to the brain.

According to this, it was confirmed that the system according to an aspect of the present invention manufactured by using cells derived from a patient can measure the metastatic potential of cancer cells in a personalized manner, and thus it was found that personalized medicine can be realized.

Overall, it was found that the system according to an aspect of the present invention had excellent effect that was able to be used to evaluate metastasis of cancer cells in other tissues in addition to the primary tumor site, to evaluate the cancer cell metastasis suppressing potential of drugs such as AMD3100, to screen cancer cell metastasis suppressing drugs, to evaluate personalized cancer cell metastatic potential by using patient-derived cancer cells, to screen a metastasis suppressing drug, and the like.

### Industrial Applicability

Embodiments of the present invention relate to a system for measuring a metastatic potential of cancer cells, a method for measuring a metastatic potential of cancer cells by using the same, a cancer cell metastasis suppressing drug screening system, and a cancer cell metastasis suppressing drug screening method. According to this, there are excellent effects of being able to easily observe and measure the metastatic potential of cancer cells and mimic the metastasis of cancer cells and measure metastatic potential thereof even when patient-derived cancer cells as well as commercialized cancer cells are applied. Further, it is possible to screen a drug for suppressing cancer cell metastasis, and thus, by using same, there is an excellent effect of realizing personalized medicine based on actual clinical practice.

## Claims

1. A system for measuring a metastatic potential of cancer cells comprising:
a tumor microenvironment mimicking unit; and
a measurement unit,
wherein the tumor microenvironment mimicking unit includes a primary tumor layer including cancer cells and vascular endothelial cells; and a metastasis layer including a gel,
wherein the vascular endothelial cells in the primary tumor layer is directed toward the metastasis layer,
wherein the cancer cells and the vascular endothelial cells are co-cultured, and
wherein the measurement unit measures a degree of migration of the cancer cells from the primary tumor layer to the metastasis layer.

2. The system for measuring a metastatic potential of cancer cells according to claim 1,
wherein, when a mimicking target of the tumor microenvironment mimicking unit is a lung microenvironment, the metastasis layer further includes fibroblasts, and
when the mimicking target of the tumor microenvironment mimicking unit is a brain microenvironment, the metastasis layer further includes astrocytes.

3. The system for measuring a metastatic potential of cancer cells according to claim 1,
wherein the tumor microenvironment mimicking unit includes two primary tumor layers, and
the metastasis layer is positioned between the two primary tumor layers.

4. The system for measuring a metastatic potential of cancer cells according to claim 1,
wherein the cancer cells of the tumor microenvironment mimicking unit is treated when a blood vessel is formed in the tumor microenvironment mimicking unit.

5. The system for measuring a metastatic potential of cancer cells according to claim 1,
wherein measurement of a degree of migration of the cancer cells is measuring an expression level of a cancer cell-specific marker.

6. The system for measuring a metastatic potential of cancer cells according to claim 5,
wherein the measurement unit performs measurement by quantifying the expression level of the cancer cell-specific marker.

7. The system for measuring a metastatic potential of cancer cells according to claim 1,
wherein the cancer cells or the vascular endothelial cells are separated from a human tissue.

8. A method for measuring a metastatic potential of cancer cells comprising:
culturing cancer cells in the primary tumor layer of the system for measuring a metastatic potential of cancer cells according to any one of claims 1 to 7; and
measuring a metastatic potential of the cancer cells after the culturing of the cancer cells.

9. A cancer cell metastasis suppressing drug screening system, comprising:
a tumor microenvironment mimicking unit; and
a measurement unit,
wherein the tumor microenvironment mimicking unit includes a primary tumor layer including cancer cells and vascular endothelial cells; and a metastasis layer including a gel,
the vascular endothelial cells in the primary tumor layer is directed toward the metastasis layer,
the cancer cells and the vascular endothelial cells are co-cultured,
the measurement unit measures a degree of migration of the cancer cells from the primary tumor layer to the metastasis layer, and
the system is treated with a cancer cell metastasis suppressing candidate.

10. The cancer cell metastasis suppressing drug screening system according to claim 9,
wherein the measurement unit measures a degree of migration of the cancer cells from the primary tumor layer to the metastasis layer in the system before and after treatment of the cancer cell metastasis suppressing candidate.

11. The cancer cell metastasis suppressing drug screening system according to claim 9,
wherein, when a degree of migration of cancer cells after the treatment of the cancer cell metastasis suppressing candidate is reduced compared to that before the treatment with the candidate, the system determines the candidate as a cancer cell metastasis suppressing drug.

12. A cancer cell metastasis suppressing drug screening method comprising:
treating the cancer cell metastasis suppressing candidate to the primary tumor layer of the cancer cell metastasis suppressing drug screening system according to any one of claims 9 to 11; and
measuring a metastatic potential of the cancer cells after the treatment of the candidate.

13. The cancer cell metastasis suppressing drug screening method according to claim 12, further comprising:
measuring a metastatic potential of the cancer cells before the treatment of the candidate; and
determining the candidate as a cancer cell metastasis suppressing drug when a degree of migration of cancer cells after the treatment of the cancer cell metastasis suppressing candidate is reduced compared to that before the treatment of the candidate.
